(19) Europäisches Patentamt
European Patent Office
Office européen des brevets

(11) Publication number: **0 535 534 A1**

# (12) EUROPEAN PATENT APPLICATION

(21) Application number: **92116370.5**

(22) Date of filing: **24.09.92**

(51) Int. Cl.[5]: **A61K 9/51**, A61K 9/16

(30) Priority: **02.10.91 US 769691**

(43) Date of publication of application: **07.04.93 Bulletin 93/14**

(84) Designated Contracting States: **DE FR GB**

(71) Applicant: **EASTMAN KODAK COMPANY**
**343 State Street**
**Rochester, New York 14650-2201(US)**

(72) Inventor: **Nair, Mridula, Eastman Kodak Company**
**Patent Legal Staff, 343 State Street**
**Rochester, New York 14650-2201(US)**

(74) Representative: **Brandes, Jürgen, Dr. rer. nat. et al**
**Wuesthoff & Wuesthoff, Patent- und Rechtsanwälte, Schweigerstrasse 2**
**W-8000 München 90 (DE)**

## (54) Biocompatible emulsion particles.

(57) The present invention relates to a particulate preparation comprising a biomedical agent associated with polymer particles having an average diameter not greater than 1 micron, and having a biocompatible hydrophilic surface derived from a biodegradable sugar based surfactant having a molecular weight of less than 2000. In another aspect, there is disclosed a process for making the particulate preparation which comprises the steps of:

(a) preparing a water immiscible organic solvent solution of a polymer, and optionally the biomedical agent;
(b) combining the solution of (a) with an aqueous solution of a biodegradable sugar based surfactant having a molecular weight of less than 2000;
(c) homogenizing the mixture formed in (b) until uniformly emulsified; and
(d) removing the organic solvent.

EP 0 535 534 A1

It is often advantageous to incorporate biomedical agents, for example, drugs or diagnostic agents, into carrier polymer nanoparticles, or microspheres, for administration into the blood stream of a host mammal rather than inject the agents themselves. The polymer particles facilitate agent solubilization, avoid rapid degradation, and toxicity, and control blood levels of the agent. However, after intravenous injection, most types of polymer particles tend to be cleared rapidly from the blood by macrophages (phagocytosis) of the reticulo-endothelial system (RES).

This is mediated by the adsorption of blood proteins onto the surfaces of the microspheres. The presence of these adsorbed proteins results in the uptake of the particles by macrophages and their rapid clearance from blood by the RES.

To deliver drugs to nonphagocytic cells, for example, to a tumor outside the RES, it is necessary to minimize competitive uptake by the RES of the drug and carrier in order to achieve prolonged circulation in the blood. Additionally, it is often necessary to be able to pass the polymer particles containing the drug across the microvasculature to the tissue/organ site in order to have adequate sequestration in the target tissue.

In this context, the size of the particles is an important parameter which governs the efficacy of the delivery system. Microsphere particles which are especially useful are nanoparticles which are small colloidal particles of less than 1 micrometer in diameter. Couvreur and others, in Polymeric Nanoparticles and Microspheres, Guiot, P. and Couvreur, P. ed., CRC Press, Boca Raton, Florida, pp. 27-93.

While emulsion polymerization offers the most direct route to small particles, the technique is limited to vinyl monomers and addition polymerizations. Further, there are potential problems in using such latices as pharmaceutical carriers, such as, (1) the nonbiodegradable nature of the polymers and (2) the possible toxicity arising from the residual monomers. Further, particles in the greater than one micrometer size range which can be made by conventional processes, while injectable, are not small enough to pass through the microvasculature to many desired tissue/organ sites. For this purpose, the size of the particles must be much smaller.

Biocompatability of particles is greatly improved by providing hydrophilic surfaces. This is usually achieved by using poly(ethylene oxide) based surfaces. A disadvantage of such surfactants is their nonbiodegradability. In addition, poly(ethylene oxide) having certain molecular weights can be toxic.

US-A-4,853,226 describes a sustained-release particulate preparation having a controlled releasing rate of a pharmaceutically active agent contained therein in the living body and a process for making the preparation. The preparation comprises a polymer which is capable of being degraded in the living body and which is compatible with living tissue, a biomedical agent, and a natural high molecular weight compound of sugar origin or a derivative thereof. The only compounds of sugar origin or a derivatives thereof which are disclosed are those having a molecular weight of greater than 2500. The preparation has "fine" and uniform particle size and can be administered subcutaneously or intramuscularly for obtaining long-lasting pharmacological activity. The particle size is said to be less than 150 micrometers. However, the process disclosed in this reference is not capable of producing particles of less than 1 micrometer in diameter.

Thus, the problem to be solved by the present invention is to provide very small particles which can pass across the microvasculature and are not sequestered rapidly by the RES. It would be additionally desirable if these particles could have sites which allow for the active targeting of the particles to specific cells/tissues/organs.

The present invention relates to a particulate preparation comprising a biomedical agent associated with polymer particles having an average diameter not greater than 1 micrometer, and having a biocompatible hydrophilic surface derived from a biodegradable sugar based surfactant having a molecular weight of less than 2000.

In another aspect of the invention, there is provided a method of preparing the above described particulate preparation, which method comprises the steps of:

(a) preparing a water immiscible organic solvent solution of a polymer and the biomedical agent;
(b) combining the solution of (a) with an aqueous solution of a biodegradable sugar based surfactant having a molecular weight of less than 2000;
(c) homogenizing the mixture formed in (b) until uniformly emulsified; and
(d) removing the organic solvent.

The advantage of the particulate preparation of this invention is that it provides extremely fine nanoparticles having a hydrophilic surface with reduced serum protein adsorption and therefore, improved blood compatibility. The particles are small enough to pass across the microvasculature. The surfactants used to provide the hydrophilic surface are biodegradable, biocompatible and nontoxic.

The particulate preparation of the present invention includes three essential components. These components are a polymer, a biomedical agent and a biodegradable sugar based surfactant. An important aspect of the invention is that the particles be less than 1 micrometer in diameter.

The particles are less than 1 micrometer so that they can cross the microvasculature. Still more preferably, they are between 0.02 and 0.5 micrometer. The particle size can be measured by photon correlation spectroscopy and transmission electron microscopy using conventional techniques.

Useful polymers are those capable of being dissolved in a solvent that is immiscible with water, and may be of any type and chemical composition, natural or synthetic, organic or inorganic, homopolymeric or random, block or graft copolymeric, amorphous or crystalline, of any tacticity, condensation or addition polymerized and/or crosslinked, thermoplastic or thermosetting, prepolymers, and of any suitable molecular weight.

Useful examples can be found in United States Patent 4,177,177 and include homo- and copolymers of olefins such as polyethylene, polypropylene, poly(propylene-co-ethylene), polyisobutylene, polyfluoroolefins, natural rubber, synthetic polyisoprenes, neoprene, polystyrene, copolymers of polystyrene with methyl methacrylate, acrylonitrile, N-vinylcarbazole or N,N-diphenylacrylamide, polyterpenes, polymers or copolymers from acrylic, methacrylic, cyano acrylic or methacrylic acids or their esters, polyacrylonitrile, vinylacetate homopolymers or copolymers with ethylene, ethyl acrylate, vinyl chloride or dibutylfumarate, homopolymers of vinyl chloride or vinylidene chloride or copolymers with each other or with acrylates or methacrylates, cellulose derivatives, such as cellulose acetate, cellulose acetate butyrate, cellulose propionate or ethyl cellulose, polyamides such as nylons 6,66,610, 11 or 12 or their copolymers, polycaprolactam, polypeptides, polyesters such as poly(ethylene terephthalate) or other polyesters of polyhydric alcohols and dicarboxylic acids, polyethers such as poly(ethylene oxide), poly(ester-amide)s, poly(ether-ester)s, polyurethanes, phosgene-bisphenol A polycarbonates, poly(amide-urethane)s, poly(ester-urethane)s, poly(carbonate-urethane)s, polyanhydrides, silicon-nitrogen, phosphorous-nitrogen or boron-nitrogen inorganic polymers, phenol, urea or melamine formaldehyde condensation polymers, poly dimethylsiloxane or other polysilicones, polysulfides and various other copolymers thereof and the like.

Particularly preferred polymers useful in this invention are biodegradable polymers such as certain polyesters, polycarbonates, or polyamides. More particularly preferred polyesters include poly(3-hydroxybutyrate-co-3-hydroxyvalerate, poly(3-hydroxybutyrate), poly(lactic acid), poly(caprolactone) and its derivatives, and poly(glycolic acid).

Biocompatible means that the material must be blood compatible and does not cause an adverse reaction in the body. For example, to be biocompatible, the material should not be toxic, immunogenic or thrombogenic. Biodegradable means that the material can be degraded either enzymatically or hydrolytically under physiological conditions to smaller molecules that can be eliminated from the body through normal processes.

Biomedical agents such as therapeutic agents, diagnostic agents, dyes or contrast agents, can be included in the particles. Particles according to the invention show little tendency towards plasma protein adsorption and subsequent macrophage uptake.

The term biomedical agent as used herein includes biologically active substances which are effective in the treatment of a physiological disorder, pharmaceuticals, enzymes, hormones, steroids, recombinant products and the like. Exemplary therapeutic agents are antibiotics, thrombolytic enzymes such as urokinase or streptokinase, insulin, growth hormone, chemotherapeutics such as adriamycin and antiviral agents such as interferon and acyclovir.

The term "diagnostic agent" as used herein includes materials which can act as contrast agents and thereby produce a detectable indicating signal in the host mammal. The detectable indicating signal may be gamma-emitting, radioactive, echogenic, fluoroscopic or physiological and the like.

The ratios of biomedical agents which are useful in the present invention are generally 50% by weight of biomedical agent to 50% by weight of the polymer, more preferably 25% by weight of biomedical agent to 75% by weight of the polymer, even more preferably, 10% by weight of biomedical agent to 90% by weight of the polymer.

The biomedical agent is associated with the polymer. By associated with, it is meant that the agent is carried by the polymer. It could be dissolved in the polymer matrix, covalently attached to the matrix or in the form of fine dispersion in the matrix.

The polymer particles of the invention have a biocompatible surface derived from a biodegradable sugar based surfactant having a molecular weight of less than 2000. Sugar based surfactants are those that are formed by attaching an oleophilic group to a sugar molecule. Particularly preferred biodegradable sugar based surfactants have a molecular weight from 400 to 800. These surface active agents are non-ionic, soluble or dispersible in water and exhibit properties that are typical of hydrophilic surfactants. For example,

they lower surface tension when in solution in water and the resulting solutions readily foam and improve the wetting of hydrophobic solid surfaces. Being non-ionic, they do not cause increases in viscosity when added to charged polyelectrolyte systems, for example aqueous gelatin. In disperse systems containing an ionic surfactant and charged polyelectrolyte, they can reduce viscosity, particularly at low shear. For a further discussion of some of the compounds useful in the context of this invention, see United States Patent 4,892,806.

More particularly, the preferred biodegradable sugar based surfactants useful in this invention include water-soluble or water-dispersible compounds having the formula:

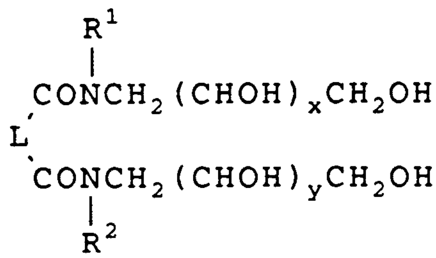

Wherein:

L is 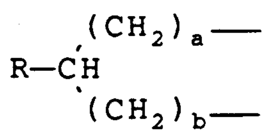 , 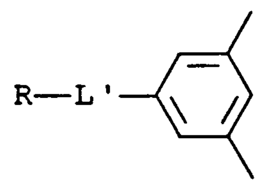 ,

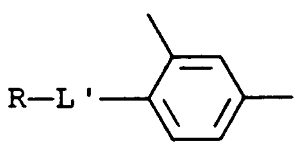 or 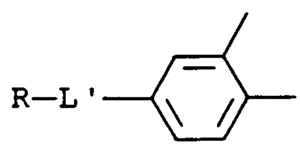 .

L' is a chemical bond, -O-, -S-, -NH-, -CONH-or $-SO_2NH-$;

R is a hydrophobic substituted or unsubstituted alkyl, substituted or unsubstituted cycloalkyl, or a substituted or unsubstituted aryl group;

each of $R^1$ and $R^2$ independently is hydrogen or an alkyl group having from 1 to 4 carbon atoms;

each of a and b independently is 0 or an integer from 1 to 3, provided that the sum of a and b is not greater than 3; and,

each of x and y independently is an integer from 3 to 7.

Preferably, R contains from 6 to 36, more preferably from 6 to 24 and most preferably from 6 to 18 carbon atoms. A particularly preferred R group is an n-alkyl group containing from 6 to 18 carbon atoms.

Preferably, each of $R^1$ and $R^2$ independently is a methyl, ethyl, propyl or butyl group.

The biodegradable sugar based surfactant "SBF-2" in the table below is an example of a surfactant of this type.

Other preferred biodegradable sugar based surfactants are described in United States patent 4,892,806. These surfactants have the structure:

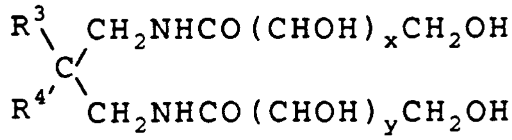

wherein: each of $R^3$ and $R^4$ independently is hydrogen, substituted alkyl, substituted or unsubstituted cycloalkyl or substituted or unsubstituted aryl provided that not both $R^3$ and $R^4$ are hydrogen; and each of x and y independently is an integer from 3 to 6.

The biodegradable sugar based surfactant "SBF-1" in the table below is an example of a surfactant of this type.

Other useful biodegradable sugar based surfactants are commercially available from Henkel Corporation and Croda Inc. See also United States Patent 3,480,616.

It is known that various intercellular recognition processes are governed largely by sugar residues on cell surfaces. The liver hepatocytes have a plasma membrane receptor for recognizing galactose; the Kupffer cells, N-acetylglucose amine; and the fibroblasts, mannose-6-phosphate. Thus, the particles of the invention, with the appropriate residues from the biodegradable sugar based surfactants, can be used for targeting to a specific cell or tissue.

In accordance with the process aspects of the invention, step (a) comprises preparing an organic solvent solution of a polymeric compound, and optionally a pharmacologically active agent. The solvent is preferably one which may be readily removed in known manner following the homogenizing step (c). The solvent system, whether a single solvent or a mixture of solvents, should be readily removed by the selected method of removal, as for example by having a boiling point below that of the water in the system.

Any suitable solvent or solvent mixture that will dissolve the polymer and optional biomedical agent and which is also immiscible with water may be used in the practice of this invention. Useful solvents include chloromethane, dichloromethane, ethyl acetate, vinyl chloride, methylethylketone (MEK), trichloromethane, carbon tetrachloride, ethylene chloride, trichloroethane, toluene, xylene, cyclohexanone, 2-nitropropane, butanol, pentanol, cyclopentanol, methyl isobutyl ketone, secondary butyl methyl ketone, diethyl ketone, ethyl isopropyl ketone, diisopropyl ketone, diethyl ether, secbutyl ether, petroleum ether, ligroin, propyl acetate, butyl and isobutyl acetate, amyl and isoamyl acetate, propyl and isopropyl propionate, ethyl butyrate, pentane, hexane, heptane, cyclopentane, cyclohexane, cycloheptane, methylene chloride, hexyl chloride, chloroform, ethylene dichloride, benzene, chlorobenzene, and mixtures thereof with each other and/or more water soluble solvents (in minor proportions).

A particularly useful solvent in the practice of this invention is dichloromethane for the reason that it is both a good solvent for many polymers while at the same time, it is immiscible with water. Further, its volatility is such that it is readily removed from the discontinuous phase droplets by evaporation.

The polymer from which the particulate preparation is made is dissolved in a quantity of solvent. The quantity of solvent is important in that the size of the particles thus prepared under given agitation conditions influences the size of the particles that result. It is generally the case that higher concentrations of polymer in the solvent produce larger particle size having a lower degree of shrinkage than that produced by lower concentrations of polymer in the same solvent. The concentration of polymer in the solvent can be from 0.5 to 50 and preferably from 0.5 to 20% by weight. The optional biomedical agent can be added to this organic solution.

In step (b) of the invention, the solution of polymer in the solvent is next introduced into an aqueous solution containing a biodegradable sugar based surfactant having a molecular weight of less than 2000. The biodegradable sugar based surfactant should be present in the aqueous phase in an amount of 0.5 to 20 times and preferably from 0.5 to 5 times as much as the polymer. The size of the particles can also be controlled to an extent by the amount of biodegradable sugar based surfactant in the aqueous phase. Generally, the higher the concentration, the smaller the particles will be.

Step (c) of the above method comprises homogenizing the mixture of (b) until emulsified. While any high shear type agitation device is useful in the process of this invention, it is preferred that the polymer in solution be introduced into the aqueous phase in a microfluidizer such as a Model No. 110T fluidizer produced by Microfluidics Manufacturing. In this device, the droplets of polymer in solvent are dispersed and reduced in size in the water phase in high shear agitation zone and upon exiting this zone, the particle size of the polymer in solution results as a discontinuous phase in the continuous aqueous phase.

As indicated, after exiting the microfluidizer, the particle size of the polymer/solvent droplets are established. The solvent is next removed from the droplets by any suitable technique, such as, heating the entire system to vaporize the solvent and thus remove it from the discontinuous phase droplets remaining in the aqueous phase. Steam distillation or other evaporative stripping methods can be used.

The resulting pseudo latices are less than one micrometer and preferably range in size from 0.01 to 0.50 micrometer, still more preferably from 0.02 to 0.20 micrometer. As noted, for parental drug delivery applications, the particle size should be no larger than 1 micrometer. The particle size of the latex can be controlled by the surfactant/polymer ratio and the concentration of the organic phase in the emulsion. The excess surfactant may be removed, if necessary, by dialysis.

In the examples which follow, the following abbreviations are used:

| SURFACTANT | STRUCTURE |
|---|---|
| SBF-1 | $[C_6H_{13}]_2C[CH_2NHCO(CHOH)_4CH_2OH]_2$ |
| SBF-2 | $C_{12}H_{25}-CH[CON(CH_3)CH_2(CHOH)_4CH_2OH]_2$ |
| Henkel APG325® Glycoside | $RO(C_6H_{10}O_5)_xH$<br>$R=C_{10}$<br>$x=1.6$ |
| Crodesta SL40 | Sucrose Monococoate<br>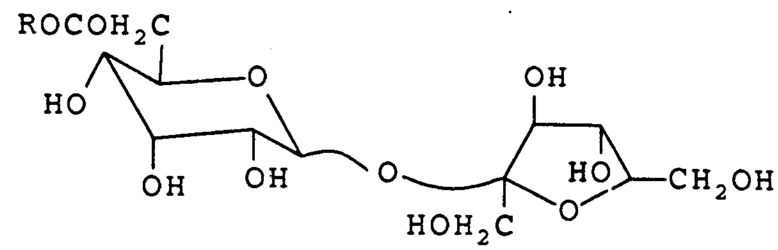<br>Where R is a fatty acid group |

Also in the examples, the steroid, available from Sterling Drug Inc., can be represented by the structure (see United States patent 3,704,295.):

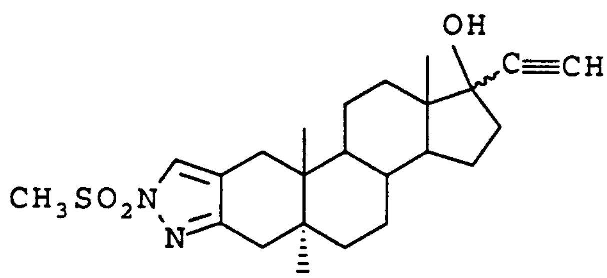

The fluorescent label was [5-N-(octadecanonyl) aminofluoroescein, available from Molecular Probes, has the structure (Referred to in the Examples as $C_{18}F$):

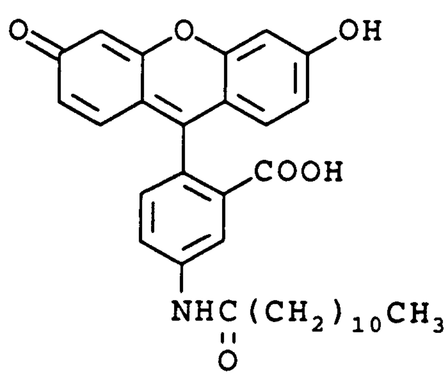

In the examples, "D69" is where n = 15 and "D70" is where n = 7 in the following formula.

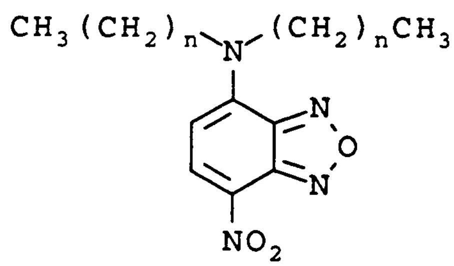

The polymers that are used in the examples below have the following abbreviations:

PHBV = poly(3-hydroxybutyrate-co-3-hydroxy-valerate), 80:20, MW-300,000
PLA = poly(L-lactic acid), MW-50,000
PCLT = poly(caprolactone)triol, MW-900
PCLD = poly(caprolactone)diol, MW-2,000
PCL = poly(caprolactone), low MW flakes
PHB = poly(3-hydroxybutryate), MW-50,000

Examples 1-17

The following detailed description is particular for Example 1 in the Table below. Other examples were prepared in an analogous manner.

A 3% solution of PHBV in dichloromethane was prepared. To this solution (36g) was added 3.5% $C_{18}F$ and 10% steroid (with respect to the polymer) in 5 mLs chloromethane. The resulting solution was mixed with SBF-1 (2g) dissolved in 400 mL distilled water using a mixer until emulsified. The emulsion was then put through a microfluidizer at 345 kPa for six passes. The dichloromethane was then removed by gentle heating. The resulting latex had a particle size of 0.055 micrometers.

In the following Table 1, "S/P" is the sugar to polymer ratio; % for the polymer is the percent in dichloromethane; % for the dye and steroid is with respect to polymer; and the size of the particles is in micrometers.

Table 1

| Ex. | Polymer (%) | Sugar (S/P) | Dye (%) | Steroid (%) | Size |
|---|---|---|---|---|---|
| 1 | PHBV (3) | SBF-1 (2:1) | $C_{18}F$ (3.5) | (10) | 0.055 |
| 2 | PHBV (3) | SBF-1 (2:1) | | | 0.054 |
| 3 | PHBV (3) | SBF-1 (1.5:1) | | | 0.073 |
| 4 | PHBV (3) | SBF-1 (1:1) | | | 0.176 |
| 4 | PHBV (3) | SBF-1 (1:1.5) | | | 0.240 |
| 6 | PHBV (3) | SBF-1 (1:3) | | | 0.360 |
| 7 | PHBV (3) | SBF-1 (2:1) | $C_{18}F$ (3.5) | | 0.049 |
| 8 | PHBV (3) | SBF-1 (2:1) | D69 (4) | | 0.085 |
| 9 | PHBV (3) | SBF-1 (2:1) | D69 (4) | (10) | 0.085 |
| 10 | PHBV (3) | SBF-1 (2:1) | D70 (4) | | 0.100 |
| 11 | PHBV (3) | SBF-1 (2:1) | D70 (4) | (10) | 0.108 |
| 12 | PHBV (3) | SBF-2 (2:1) | $C_{18}F$ (4) | | 0.055 |
| 13 | PHBV (3) | SBF-2 (2:1) | $C_{18}F$ (4) | (10) | 0.054 |
| 14 | PHBV (3) | Henkel (2:1) | $C_{18}F$ (4) | | 0.052 |
| 15 | PHBV (3) | Crodesta (2:1) | $C_{18}F$ (4) | | 0.067 |
| 16 | PCL (3) | SBF-1 (2:1) | $C_{18}F$ (4) | | 0.096 |
| 17 | PLA (3) | SBF-1 (2:1) | $C_{18}F$ (4) | | 0.041 |

Example 18:

The effect of the biodegradable sugar based surfactant on serum protein adsorption using polystyrene particles was examined. The polystyrene particles were made by emulsion polymerization. Biodegradable sugar based surfactants were adsorbed onto the particles by incubation of the particles in an aqueous solution of the surfactant. The particles were less than 0.5 micrometers. Protein adsorption was estimated by recovering the particles from serum after incubation, washing with phosphate buffered saline, releasing the adsorbed protein with sodium dodecyl sulfate, and analyzing by gel electrophoresis. The biodegradable sugar based surfactants SBF-1, SBF-2 and Henkel APG325® successfully inhibit protein adsorption from human serum for periods of up to 2 hours compared with styrene particles alone.

Example 19:

*In vitro* studies were conducted to determine the biological behavior of the particles according to the invention. Peritoneal macrophages were incubated in the presence of the $C_{18}F$ labeled particies for a 24 hour period. At the end of this period, the cells were harvested and the amount of the fluorescence associated with the cells was determined with a fluorimeter.

Three controls were prepared. Two controls used polystyrene (PS) particles of two different average diameters alone. The third control used PS particles with a poly(ethylene oxide) (PEO) based surfactant (Pluronic®) treatment as is known in the art. The results are given in the following Table 2:

Table 2

| Particles | Size | Phagocytic uptake particles/cell |
| --- | --- | --- |
| PS | 0.050 | 107 |
| PS | 0.176 | 177 |
| PS + PEO | 0.176 | <10 |
| HBV + SBF-1 + C18F + steroid | 0.095 | <10 |
| HBV + SBF-1 + C18F | 0.054 | 33 |
| HBV + Henkel + C18F | 0.052 | 28 |
| HBV + Crodesta + C18F | 0.067 | 13 |

These results show that the particles of the invention show significant supression of phagocytosis and compare well with PEO treated particles but have none of the disadvantages of PEO such as potential toxicity and nonbiodegradability.

## Claims

1. A particulate preparation comprising a biomedical agent associated with polymer particles having an average diameter not greater than 1 micron, and having a biocompatible hydrophilic surface derived from a biodegradable sugar based surfactant having a molecular weight of less than 2000.

2. A particulate preparation as claimed in claim 1, wherein said polymer is selected from the group consisting of poly(3-hydroxybutyrate-co-3-hydroxyvalerate, poly(3-hydroxybutyrate), poly(lactic acid), poly(caprolactone) and its derivatives, and poly(glycolic acid).

3. A particulate preparation as claimed in claim 1, wherein said biodegradable sugar based surfactant has the formula:

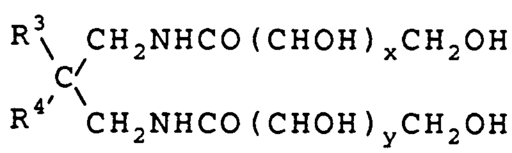

wherein: each of $R^3$ and $R^4$ independently is hydrogen, substituted alkyl, substituted or unsubstituted cycloalkyl, or substituted or unsubstituted aryl provided that not both $R^3$ and $R^4$ are hydrogen; and each of x and y independently is an integer from 3 to 6.

4. A particulate preparation as claimed in claim 3 wherein $R^3$ is $C_6H_{13}$ and $R^4$ is $C_6H_{13}$.

5. A particulate preparation as claimed in claim 3 having the following formula:

$$[C_6H_{13}]_2C[CH_2NHCO(CHOH)_4CH_2OH]_2$$

6. A particulate preparation as claimed in claim 1 wherein said biodegradable sugar based surfactant has the formula:

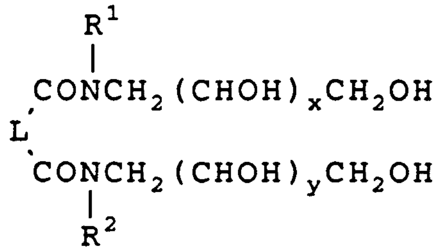

Wherein:

L is 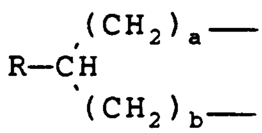 , 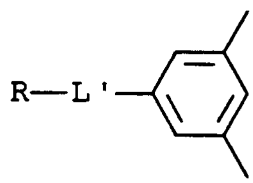 ,

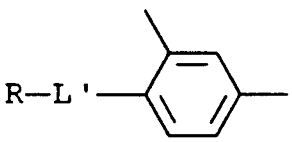 or 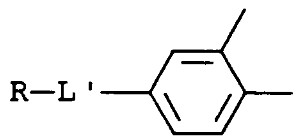 .

L' is a chemical bond, -O-, -S-, -NH-, -CONH-or $-SO_2NH-$;

R is a hydrophobic substituted or unsubstituted alkyl, substituted or unsubstituted cycloalkyl, or a substituted or unsubstituted aryl group;

each of $R^1$ and $R^2$ independently is hydrogen or an alkyl group having from 1 to 4 carbon atoms;

each of a and b independently is 0 or an integer from 1 to 3, provided that the sum of a and b is not greater than 3; and,

each of x and y independently is an integer from 3 to 7.

**7.** A particulate preparation as claimed in claim 6 wherein said biodegradable sugar based surfactant is represented by the formula:

$$C_{12}H_{25}-CH-[CON(CH_3)CH_2(CHOH)_4CH_2OH]_2$$

**8.** A method of preparing a particulate preparation comprising a biomedical agent associated with polymer particles having an average diameter not greater than 1 micron, and having a biocompatible hydrophilic surface derived from a biodegradable sugar based surfactant having a molecular weight of less than 2000, which method comprises the steps of:

(a) preparing a water immiscible organic solvent solution of a polymer, and optionally the biomedical agent;

(b) combining the solution of (a) with an aqueous solution of a biodegradable sugar based surfactant having a molecular weight of less than 2000;

(c) homogenizing the mixture formed in (b) until uniformly emulsified; and

(d) removing the organic solvent.

European Patent Office

# EUROPEAN SEARCH REPORT

Application Number

EP 92 11 6370

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | WO-A-9 115 193 (RHONE-POULENC RORER) 17 October 1991<br>* page 2, line 22 - line 25 *<br>* page 3, line 9 - line 20 *<br>* page 3, line 24 - line 34 *<br>* page 4, line 8 - line 26 *<br>* page 8; example 12 *<br>* claims 1-6 *<br>--- | 1,2,8 | A61K9/51<br>A61K9/16 |
| X | INT. J. PHARM.<br>vol. 61, no. 1,2, 11 June 1990,<br>pages 237 - 246<br>MULLER R.H. ET AL 'IN VITRO CHARACTERIZATION OF POLY(METHYL-METHACRYLATE) NANOPARTICLES AND CORRELATION TO THEIR IN VIVO FATE'<br>*page 238-239,"Materials and method"*<br>* page 239, paragraph 4 - page 241 *<br>--- | 1,2 | |
| A | WO-A-9 011 069 (PARFUMS CHRISTIAN DIOR) 4 October 1990<br>* page 18 - page 19; example 5 *<br>--- | 1 | TECHNICAL FIELDS SEARCHED (Int. Cl.5 ) |
| A | EP-A-0 274 961 (CENTRE NATIONAL DE LA RECHERCHE SCIENTIFIQUE)<br>20 July 1988<br>--- | | A61K |
| D,A | US-A-4 892 806 (BRIGGS C.B.A. ET AL)<br>9 January 1990<br>* column 6 - column 7; example 2 *<br>* claim 1 *<br>----- | 3-7 | |

The present search report has been drawn up for all claims

| Place of search | Date of completion of the search | Examiner |
|---|---|---|
| THE HAGUE | 13 JANUARY 1993 | BOULOIS D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)